# EUROPEAN PATENT APPLICATION

(11) **EP 4 588 941 A1**
(43) Date of publication of application: **23.07.2025**
(21) Application number: 23864826.5
(22) Date of filing: 18.09.2023
(51) Int. Cl.: C07K 19/00, C12N 15/62, C12N 5/10, A61K 47/64, A61K 47/68, A61P 35/00, A61P 9/04, A61P 37/02

(54) **NKG2D-NKP46 CELL ADAPTER MOLECULE AND USE THEREOF**

(30) Priority: 16.09.2022 CN 202211130558
(71) Applicant: West China Hospital of Sichuan University, Chengdu, Sichuan 610041 (CN)
(72) Inventor: ZHAO, Xudong, Chengdu, Sichuan 610041 (CN); YANG, Dong, Chengdu, Sichuan 610041 (CN)
(74) Representative: Santoro, Sofia
(86) International application number: PCT/CN2023/119477
(87) International publication number: WO 2024/056098

(57) **Abstract**

Provided are an NK cell adapter molecule targeting an NKG2D ligand and an NKp46 and the use thereof. The cell adapter molecule at least comprises: a first binding domain comprising an NKG2D extracellular domain that can specifically bind to the NKG2D ligand; and a second binding domain comprising an NKp46 antigen-binding fragment. The cell adapter molecule can promote the killing effect of natural killer cells on target cells with high levels of expression of NKG2D ligand, and can thus be used as a therapeutic agent for diseases related to NKG2D ligand expression.

## Description

### Technical Field

The present invention relates to the field of biomedicine, and particularly relates to a cell engager molecule consisting of an NKG2D extracellular domain capable of specifically binding to a NKG2D ligand, a linker segment, and an NKp46 antigen-binding fragment, and the application thereof.

### Background

NKG2D is expressed on the surface of NK cells, CD8⁺ T cells, activated macrophages, and tumor-infiltrating γδ T cells, typically existing as a self-formed homodimer. It directly activates NK cells through the adaptor proteins DAP10 or DAP12 to exert cytotoxic effects or acts as a co-stimulatory signal to promote T cell activation. Its ligands mainly include MICA, MICB, and ULBP1-6, which are generally not expressed or are expressed at low levels in normal cells. However, when cells are under stress conditions such as abnormal transformation, viral infection, or DNA damage, the expression of NKG2D ligands can be significantly upregulated, serving as markers of abnormal cells for recognition by the immune system. The NKG2D-NKG2D ligand signaling chain is a crucial mechanism for the immune surveillance function of the body's immune system. Immunotherapy strategies targeting this pathway are expected to have better safety and efficacy and have gradually become a frontier area in current immunotherapy approaches To date, more than 10 CAR-T and CAR-NK therapies designed based on the NKG2D-NKG2DL mechanism have entered clinical studies, with no severe treatment-related adverse events reported. Cell engager molecules constructed based on the NKG2D extracellular domain have also gained increasing attention in the field of anti-tumor therapy. For example, the T cell bispecific antibody NKG2D-CD3 can inhibit tumor growth and prolong the survival of model mice *in vivo* by targeting tumor cells and immunosuppressive cells. The anti-NK cell bispecific antibody NKG2D-CD 16 can efficiently kill tumor cells such as acute myeloid leukemia, lymphoma, and soft tissue sarcoma; NKG2D-Fc can significantly improve the tumor immune microenvironment and inhibit tumorigenesis.

Currently, there is still a need in this field to develop more cell engager molecules.

### Summary of the invention

The purpose of the present invention is to provide a cell engager molecule comprising an NKG2D extracellular domain and an NKp46 antigen-binding fragment and uses thereof.

In the first aspect of the present invention, it provides a cell engager molecule comprising:
(a) a first binding domain that specifically binds to a NKG2D ligand; and
(b) a second binding domain that specifically binds to NKp46.

In another preferred embodiment, the first binding domain is derived from an extracellular domain of NKG2D.

In another preferred embodiment, the amino acid sequence of the first binding domain is as shown in SEQ ID NO: 1; or is an amino acid sequence that has at least 80%, 85%, 90%, 91%, 92%, 93%, 94%, 95%, 96%, 97%, 98%, or 99% sequence homology or sequence identity to the amino acid sequence as shown in SEQ ID NO: 1 and is capable of binding to a NKG2D ligand.

In another preferred embodiment, the second binding domain comprises an antigen-binding fragment specifically against NKp46.

In another preferred embodiment, the antigen-binding fragment against NKp46 comprises a structure selected from the group consisting of: Fab, Fab', F(ab')2, Fd, Fv, dAb, Fc, complementarity-determining region (CDR) fragment, single-chain antibody, single-domain antibody, and a combination thereof.

In another preferred embodiment, the antigen-binding fragment against NKp46 comprises a structure selected from the group consisting of: a humanized antibody, a chimeric antibody, and a combination thereof.

In another preferred embodiment, the antigen-binding fragment against NKp46 comprises: an Fab fragment, a single-chain antibody (scFv), a single-domain antibody, and a combination thereof.

In another preferred embodiment, the Fab fragment and/or single-chain antibody against NKp46 comprises a heavy chain variable region and a light chain variable region, wherein the heavy chain variable region comprises the following heavy chain complementarity-determining regions (HCDRs):
1) HCDR1 with the amino acid sequence as shown in SEQ ID NO: 3;
2) HCDR2 with the amino acid sequence as shown in SEQ ID NO: 4;
3) HCDR3 with the amino acid sequence as shown in SEQ ID NO: 5; and

the light chain variable region comprises the following light chain complementarity determining regions (CDRs):
   1) LCDR1 with the amino acid sequence as shown in SEQ ID NO: 6;
   2) LCDR2 with the amino acid sequence YTS;
   3) LCDR3 with the amino acid sequence as shown in SEQ ID NO: 7;
wherein the CDRs are based on the IMGT numbering and definition scheme.

In another preferred embodiment, the heavy chain variable region of the anti-NKp46 Fab fragment or scFv has an amino acid sequence as shown in SEQ ID NO: 8, or an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 8.

In another preferred embodiment, the light chain variable region of the anti-NKp46 Fab fragment or scFv has an amino acid sequence as shown in SEQ ID NO: 9, or an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 9.

In another preferred embodiment, the anti-NKp46 Fab fragment has an amino acid sequence as shown in SEQ ID NO: 2, or an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 2 (the sequence is cited from the reference Gauthier, Morel et al. 2019).

In another preferred embodiment, the anti-NKp46 scFv fragment has an amino acid sequence as shown in SEQ ID NO: 10, or an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 10 (the sequence is cited from the reference Gauthier, Morel et al. 2019).

In another preferred embodiment, the heavy chain complementarity-determining regions of the anti-NKp46 single-domain antibody comprises:
1) HCDR1 with the amino acid sequence as shown in SEQ ID NO: 3;
2) HCDR2 with the amino acid sequence as shown in SEQ ID NO: 4;
3) HCDR3 with the amino acid sequence as shown in SEQ ID NO: 5.

In another preferred embodiment, the anti-NKp46 single-domain antibody has an amino acid sequence as shown in SEQ ID NO: 11, or has an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 11.

In another preferred embodiment, the second binding domain further comprises: an antigen-binding fragment derived from an anti-CD16 antibody and/or a polypeptide of an IL 15 protein.

In another preferred embodiment, the antigen-binding fragment targeting CD16 comprises a structure selected from the group consisting of: Fab, Fab', F(ab')2, Fd, Fv, dAb, Fc, complementarity-determining region (CDR) fragment, single-chain antibody, single-domain antibody, and a combination thereof.

In another preferred embodiment, the antigen-binding fragment against NKp46 comprises a structure selected from the group consisting of: a humanized antibody, a chimeric antibody, and a combination thereof.

In another preferred embodiment, the variable region of the anti-CD16 single-domain antibody comprises:
1) HCDR1 with the amino acid sequence as shown in SEQ ID NO: 12;
2) HCDR2 with the amino acid sequence as shown in SEQ ID NO: 13;
3) HCDR3 with the amino acid sequence as shown in SEQ ID NO: 14.

In another preferred embodiment, the anti-CD16 single-domain antibody has an amino acid sequence as shown in SEQ ID NO: 15, or has an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 15.

In another preferred embodiment, the anti-CD16 Fc fragment has an amino acid sequence as shown in SEQ ID NO: 16, or an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 16.

In another preferred embodiment, the IL 15 protein has an amino acid sequence as shown in SEQ ID NO: 17, or has an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 17.

In another preferred embodiment, the cell engager molecule has a structure selected from the following formula (I) or (II) from N-terminus to C-terminus:

S-D₁-L₁-D₂-T (I);

or

S-D₂-L₁-D₁-T (II)

, wherein,
each "-" is independently a linking peptide or peptide bond;
S is absent or a signal peptide sequence;
D₁ is the first binding domain;
L₁ is absent, a first linker peptide, or a single-chain Fc (scFc) fragment;
D₂ is the second binding domain;
T is absent or a marker protein.

In another preferred embodiment, S is a signal peptide derived from mammalian CD8α.

In another preferred embodiment, the first linker peptide is a flexible linker peptide or a rigid linker peptide.

In another preferred embodiment, the first linker peptide is a glycine-serine linker peptide.

In another preferred embodiment, the first linker peptide is represented by the formula (G4S)n, wherein n is 1, 2, 3, 4, 5, or 6, and preferably n is 3.

In another preferred embodiment, the single-chain Fc fragment is formed by two Fc monomers connected via a linker.

In another preferred embodiment, the amino acid sequence of the single-chain Fc fragment is as shown in SEQ ID NO: 18.

In another preferred embodiment, the second binding domain has a structure selected from the following formula (III) or (IV) from the N-terminus to the C-terminus:

D₃-L₂-D₄-I (III);

or

D₄-L₂-D₃- I (IV),

wherein,
each "-" is independently a linking peptide or peptide bond;
D3 is an NKp46 antigen-binding fragment;
L2 is absent or a second linker peptide;
D4 is absent or a CD16 antigen-binding fragment;
I is absent or an IL15 protein.

In another preferred embodiment, the second linker peptide is a glycine-serine linker peptide.

In another preferred embodiment, the second linker peptide is represented by the formula (G4S)n, wherein n is 1, 2, 3, 4, 5, or 6, and preferably n is 3.

In another preferred embodiment, the marker protein T is selected from: His tag, FLAG tag.

In the second aspect of the present invention, it provides a recombinant protein comprising the cell engager molecule according to the first aspect of the present invention.

In another preferred embodiment, the recombinant protein (or polypeptide) comprises a fusion protein.

In another preferred embodiment, the recombinant protein specifically binds to an NKG2D ligand and an NKp46 antigen on the surface of NK cell.

In another preferred embodiment, the recombinant protein further specifically binds to CD16.

In a third aspect of the present invention, it provides a polynucleotide, which encodes a polypeptide selected from the group consisting of:
(1) the cell engager molecule according to the first aspect of the present invention; or
(2) the recombinant protein according to the second aspect of the present invention.

In the fourth aspect of the present invention, it provides a vector, which comprises the polynucleotide according to the third aspect of the present invention.

In another preferred embodiment, the vector includes but is not limited to: bacterial plasmid, phage, yeast plasmid, plant cell virus, mammalian cell virus such as adenovirus, retrovirus, or other vectors.

In another preferred embodiment, the vector includes but is not limited to: pCDH, pTOMO, pGEM, pELNS, pMSGV, and a combination thereof.

In the fifth aspect of the present invention, it provides a engineered host cell, wherein the host cell comprises the vector according to the fourth aspect of the present invention or has the polynucleotide according to the third aspect of the present invention integrated into its genome.

In another preferred embodiment, the host cell is an *in vivo* cell or an *in vitro* cultured cell that can be transplanted into the body.

In another preferred embodiment, the immune cell is selected from a NK cell.

In another preferred embodiment, the immune cell is derived from human or a non-human mammal (such as a mouse).

In the sixth aspect of the present invention, it provides an antibody conjugate which comprises:
(a) an antibody moiety, which is selected from the group consisting of the cell engager molecule according to the first aspect of the present invention; and
(b) a coupling moiety coupled to the antibody moiety, which is selected from the group consisting of a detectable label, a drug, and a combination thereof.

In another preferred embodiment, the detectable marker comprises a radionuclide.

In another preferred embodiment, the drug includes a toxin, a cytokine, or an enzyme.

In another preferred embodiment, the coupling moiety is selected from the group consisting of a fluorescent or luminescent marker, a radioactive marker, MRI (magnetic resonance imaging) or CT (electronic computer X-ray tomography technique) contrast agent, or an enzyme capable of producing a detectable product, a radionuclide, a biotoxin, a cytokine (such as IL-2, etc.), an antibody, an Fc fragment of an antibody, an scFv fragment of an antibody, a gold nanoparticle/nanorod, a viral particle, a liposome, a magnetic nanoparticle, a prodrug activating enzyme (such as DT-diaphorase (DTD) or biphenyl hydrolase-like protein (BPHL)), chemotherapeutic agent (such as cisplatin) and any form of nanoparticles, and the like.

In another preferred embodiment, the antibody moiety and the coupling moiety are coupled through a chemical bond or a linker.

In another preferred embodiment, the immunoconjugate comprises a multivalent (e.g., bivalent) cell engager molecule according to the first aspect of the present invention.

In another preferred embodiment, the multivalency refers to the inclusion of multiple repeats of the cell engager molecule according to the first aspect of the present invention in the amino acid sequence of the immunoconjugate.

In the seventh aspect of the present invention, it provides a pharmaceutical composition which comprises:
(a) an active ingredient selected from the group consisting of: the cell engager molecule according to the first aspect of the present invention, the recombinant protein according to the second aspect of the present invention, the host cell according to the fifth aspect of the present invention, the antibody conjugate according to the sixth aspect of the present invention, and a combination thereof; and
(b) one or more pharmaceutically acceptable carriers, diluents, fillers, binders, excipients, or a combination thereof.

In another preferred embodiment, the pharmaceutical composition is a liquid preparation.

In another preferred embodiment, the pharmaceutical composition is an injection.

In another preferred embodiment, the pharmaceutical composition comprises 0.01~99.99% of the cell engager molecule according to the first aspect of the present invention, the recombinant protein according to the second aspect of the present invention, the host cell according to the fifth aspect of the present invention, the antibody conjugate according to the sixth aspect of the present invention, or a combination thereof, and 0.01~99.99% of a carrier, wherein the percentages are mass percentages relative to the pharmaceutical composition.

In another preferred embodiment, the pharmaceutical composition is used for preventing and/or treating a disease with upregulated NKG2D ligand expression.

In the eighth aspect of the present invention, it provides a use of the cell engager molecule according to the first aspect of the present invention, the recombinant protein according to the second aspect of the present invention, the host cell according to the fifth aspect of the present invention, the antibody conjugate according to the sixth aspect of the present invention, and/or the pharmaceutical composition according to the seventh aspect of the present invention in the preparation of a medicament for treating a disease associated with upregulated NKG2D ligand expression.

In another preferred embodiment, the medicament is used for preventing and/or treating a disease.

In another preferred embodiment, the upregulation means that the ratio of the expression level of NKG2D ligand (F1) to that in normal cells and tissues (F0) (i.e., F1/F0) is ≥1.5, preferably ≥2, more preferably ≥2.5.

In another preferred embodiment, the NKG2D ligand includes (but is not limited to) MICA, MICB, ULBP1, ULBP2, ULBP3, ULBP4, ULBP5, ULBP6.

In another preferred embodiment, the disease includes tumors, autoimmune diseases, transplant rejection, inflammation, aging, and diseases associated with senescent cell accumulation.

In another preferred embodiment, the tumor includes hematological tumor and solid tumor.

In another preferred embodiment, the hematological tumor is selected from a group consisting of: acute myeloid leukemia (AML), multiple myeloma (MM), chronic lymphocytic leukemia (CLL), acute lymphocytic leukemia (ALL), diffuse large B cell lymphoma (DLBCL) and a combination thereof.

In another preferred embodiment, the solid tumor is selected from the group consisting of: lung cancer, ovarian cancer, colorectal cancer, liver cancer, gallbladder cancer, biliary tract cancer, gastric cancer, pancreatic cancer, kidney cancer, prostate cancer, breast cancer, bladder cancer, nasopharyngeal cancer, non-small cell lung cancer, glioblastoma, neuroblastoma, melanoma, and a combination thereof.

In another preferred embodiment, the disease associated with senescent cell accumulation is selected from the group consisting of: muscular atrophy, fatty liver, heart failure, atherosclerosis, diabetes, myocardial hypertrophy, osteoporosis, tissue/organ fibrosis, Alzheimer's disease, Parkinson's syndrome, arthritis, chronic obstructive pulmonary disease, and other degenerative diseases caused by cellular senescence, and a combination thereof.

In another preferred embodiment, the medicament is used for inhibiting cells with upregulated NKG2D ligand expression, preferably including: human liver cancer cell line MHCC97H, human liver cancer cell line SMMC7721, human pancreatic cancer cell line ASPC1, or a combination thereof.

In another preferred embodiment, the senescent cells are selected from the group consisting of: lung cells, fat cells, kidney cells, muscle cells, and a combination thereof.

In another preferred embodiment, the senescent cell is the human embryonic lung cell line HEL1.

In another preferred embodiment, the senescent cell is naturally occurring or artificially induced.

In another preferred embodiment, the artificial senescence induction method includes: senescence induction via DNA damage, senescence induction via P16 overexpression, senescence induction via telomere shortening, and a combination thereof.

In another preferred embodiment, the autoimmune disease is selected from the group consisting of: rheumatoid arthritis, colitis, celiac disease, multiple sclerosis, alopecia areata, type 1 diabetes, chronic obstructive pulmonary disease, atherosclerosis, and metabolic syndrome associated with type 2 diabetes.

In another preferred embodiment, the method further comprises treating the subject with an additional disease treatment method.

In another preferred embodiment, the additional disease treatment method is selected from the group consisting of: surgery, radiation therapy, chemotherapy, gene therapy, DNA therapy, viral therapy, RNA therapy, adjuvant therapy, immunotherapy, and a combination thereof.

In the ninth aspect of the present invention, it provides a method for treating a disease associated with upregulated NKG2D ligand expression, comprising administering an effective amount of the cell engager molecule according to the first aspect of the present invention, the recombinant protein according to the second aspect of the present invention, the host cell according to the fifth aspect of the present invention, the antibody conjugate according to the sixth aspect of the present invention, the pharmaceutical composition according to the seventh aspect of the present invention, or a combination thereof to a subject in need thereof.

It should be understood that, within the scope of the present invention, the technical features specifically described above and below (such as the Examples) can be combined with each other, thereby constituting a new or preferred technical solution which needs not be described one by one.

### DESCRIPTION OF DRAWINGS

The following drawings are used to illustrate specific embodiments of the present invention and are not intended to limit the scope of the present invention as defined by the claims.

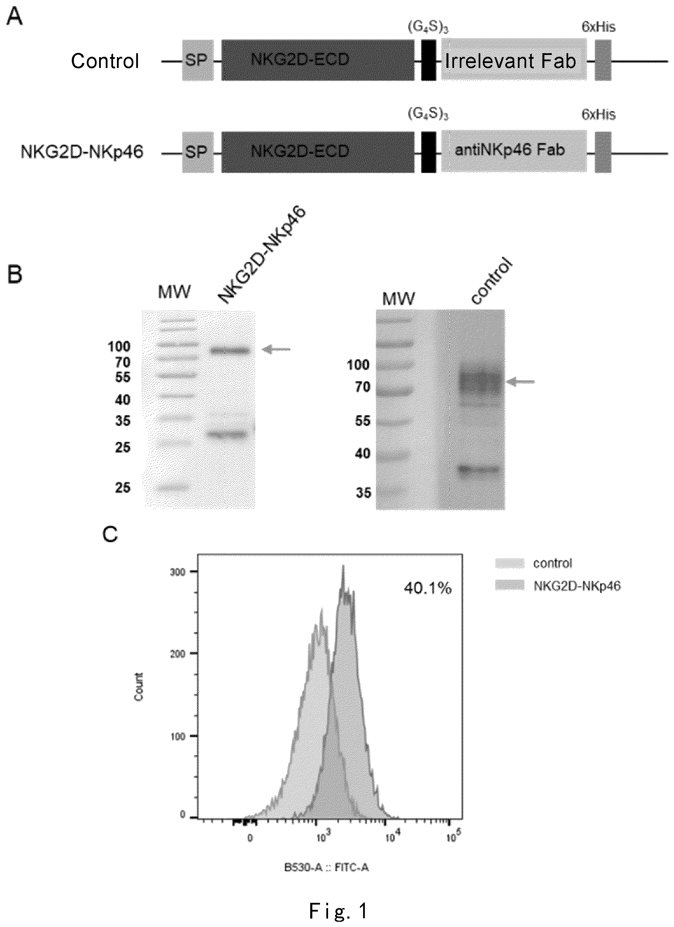

Figure 1 shows the preparation of the NKG2D-NKp46 protein. (A) Schematic diagram of the NKG2D-NKp46 and control vector structures. (B) After transfection of 293T cells with NKG2D-NKp46 and control vectors, the culture supernatant was collected and purified. The expression of NKG2D-NKp46 and control proteins in the purified samples was detected using an anti-His antibody. (C) After incubation of NKG2D-NKp46 with NK cells, the binding rate was detected by flow cytometry.

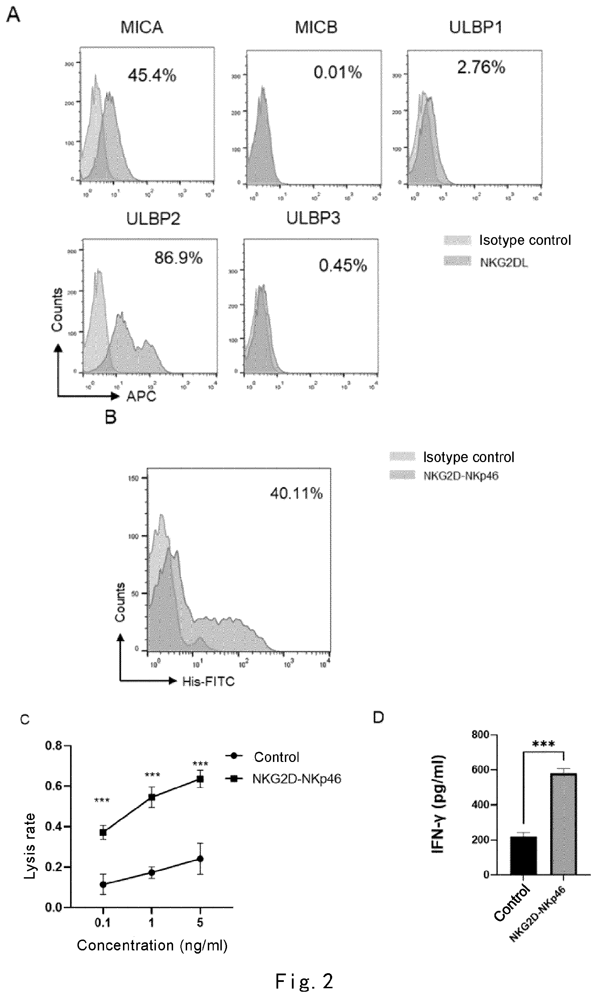

Figure 2 shows that the NKG2D-NKp46 protein promotes NK cell-mediated killing of tumor cells. (A) The expression of NKG2D ligands MICA, MICB, ULBP1, ULBP2, and ULBP3 in the liver cancer cell line MHCC97H was detected by flow cytometry. (B) The binding rate was detected by flow cytometry after co-incubation of NKG2D-NKp46 with MHCC97H cells. (C) The mortality rate of MHCC97H cells was measured 8 hours after adding the NKG2D-NKp46 protein to the co-culture system of NK cells and MHCC97H cells. (D) The expression of IFN-γ was detected by enzyme-linked immunosorbent assay (ELISA) after adding the NKG2D-NKp46 protein to the co-culture system of NK cells and MHCC97H cells for 8 hours.

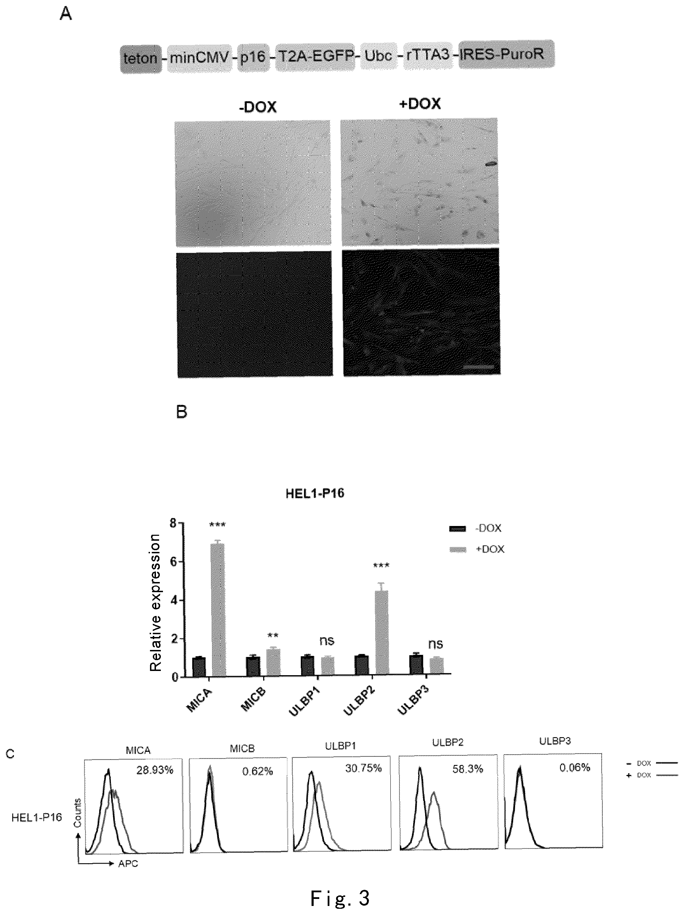

Figure 3 shows the upregulation of NKG2D ligand expression in senescent cells. (A) and then subjected to β-gal staining. (B) HEL1-P16 cells were induced to overexpress P16 using tetracycline (DOX), and the expression of NKG2D ligands was detected by quantitative PCR. (C) HEL1-P16 cells were induced to overexpress P16 using tetracycline (DOX), and the expression of NKG2D ligands was detected by flow cytometry.

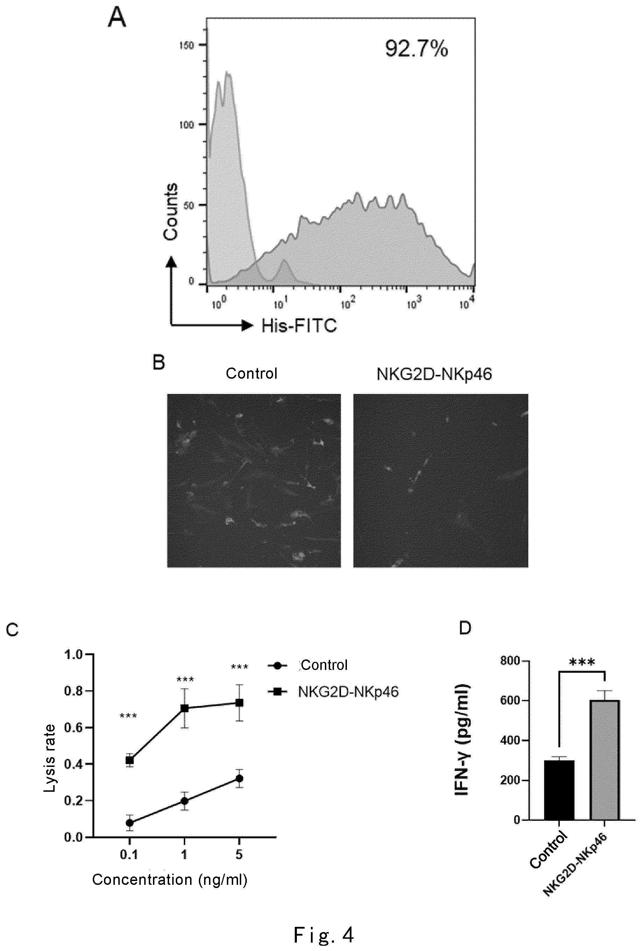

Figure 4 shows that the NKG2D-NKp46 protein promotes NK cell-mediated killing of senescent cells. (A) After co-incubation of NKG2D-NKp46 with senescent cells, the binding rate was detected by flow cytometry. (B) The mortality rate of senescent cells was measured using microscopy 8 hours after adding the NKG2D-NKp46 protein to the co-culture system of NK cells and DOX-induced senescent HEL1-P16 cells. (C) The lysis rate of senescent cells was detected after adding different concentrations of the NKG2D-NKp46 protein to the co-culture system of NK cells and DOX-induced senescent HEL1-P16 cells. (D) The expression of IFN-γ was detected by enzyme-linked immunosorbent assay (ELISA) after adding the NKG2D-NKp46 protein (5 µg/ml) to the co-culture system of NK cells and senescent HEL1-P16 cells for 8 hours.

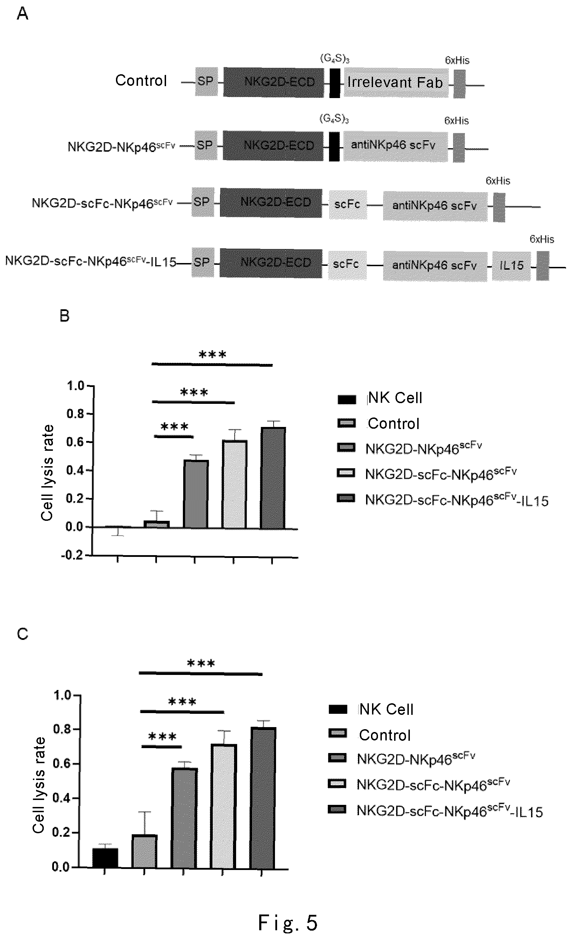

Figure 5 shows that the protein comprising NKp46 scFv promotes NK cell-mediated killing. (A) Schematic diagram of the structure of the protein comprising NKp46 scFv and the control vector. The structure of scFc is: CH2-CH3-CH2-CH3. CH2 and CH3 are derived from human IgG1 antibody. NKp46 scFv is the anti-NKp46 single-chain antibody. (B) The lysis rate of SMMC7721 cells was detected 24 hours after adding the protein comprising NKp46 scFv to the co-culture system of resting NK cells and SMMC7721 cells. (C) The lysis rate of cells was detected 24 hours after adding the protein comprising NKp46 scFv to the co-culture system of resting NK cells and DOX-induced senescent HEL1-P16 cells.

### EMBODIMENTS

Through extensive and intensive research and a large amount of screening, the inventors of the present invention have, for the first time, developed the preparation and application of a bispecific cell engager molecule based on the extracellular domain of NKG2D. Experimental results demonstrate that the bispecific antibody targeting NKG2D ligands and NKp46 antigen according to the present invention exhibits significant killing effects on target cells. On this basis, the present invention has been completed.

The bispecific cell engager molecule provided by the present invention consists of three parts: the extracellular domain of NKG2D that specifically binds to NKG2D ligands, a linker segment, and a NKp46 antigen-binding fragment. The bispecific cell engager can bridge natural killer cells to the vicinity of target cells with high expression of NKG2D ligands, thereby killing the target cells. Thus, it can be used to treat diseases associated with the expression of NKG2D ligands.

### Terms

To make it easier to understand the present invention, certain technical and scientific terms are specifically defined below. Unless otherwise clearly defined herein, all other technical and scientific terms used herein have meanings commonly understood by those skilled in the art in the field to which the present invention belongs. Before describing the present invention, it should be understood that the present invention is not limited to the specific methods and experimental conditions described, as such methods and conditions can be changed. It should also be understood that the terms used herein are only intended to describe the specific embodiments and are not intended to be restrictive. The scope of the present invention will be limited only by the accompanying claims.

Unless otherwise defined, all technical and scientific terms used herein have the same meaning as would normally be understood by those of ordinary skill in the art to which the invention belongs. As used herein, term "about" means that the value may change by no more than 1% from the enumerated value when used in reference to a specific enumerated value. For example, as used herein, the expression "about 100" includes all values between 99 and 101 and (e. g., 99.1, 99.2, 99.3, 99.4, etc.).

The three-letter and single-letter amino acid codes used in the present invention are as described in J. Biol. Chem., 243, p3558 (1968).

As used herein, the term "treatment" refers to the administration of an internal or external therapeutic agent, including an antibody against the respiratory syncytial virus fusion protein (preferably fused pre-F protein) of the present invention and its composition and composition thereof of the present invention, to a patient exhibiting one or more disease symptoms, wherein the therapeutic agent is known to have a therapeutic effect on these symptoms. Typically, the therapeutic agent is administered in an amount sufficient to alleviate one or more disease symptoms (a therapeutically effective amount).

As used herein, the term "optional" or "optionally" means that the event or situation described thereafter may occur, but not necessarily. For example, "optionally comprising 1-3 antibody heavy chain variable regions" means that the antibody heavy chain variable region of a specific sequence may be comprised but does not have to be comprised, and the number of it can be 1, 2 or 3.

The "sequence identity" described in the present invention refers to the degree of identity between two nucleic acid sequences or two amino acid sequences with appropriate mutations such as substitutions, insertions, or deletions when optimally aligned and compared. The sequence identity between a sequence described in the present invention and a sequence having identity thereto may be at least 85%, 90%, or 95%, preferably at least 95%. Non-limiting examples include 85%, 86%, 87%, 88%, 89%, 90%, 91%, 92%, 93%, 94%, 95%, 96%, 97%, 98%, 99%, and 100%.

### NKG2D and NKG2D Ligand

NKG2D is expressed on the surface of NK cells, CD8+ T cells, activated macrophages, and tumor-infiltrating γδ T cells. It can directly activate NK cells to exert cytotoxic effects or serve as a co-stimulatory signal to promote T cell activation.

The present invention constructs a cell engager molecule that binds to NKG2D ligands based on the extracellular domain of NKG2D. The NKG2D ligand described in the present invention includes (but is not limited to) MICA, MICB, ULBP1, ULBP2, ULBP3, ULBP4, ULBP5, and ULBP6.

### NKp46

NKp46 belongs to the immunoglobulin superfamily and has two Ig-like domains. It is the primary activating receptor for NK cells to eliminate target cells *in vivo.* Antibodies targeting NKp46 can effectively trigger NK cell cytotoxicity and cytokine release.

The present invention constructs an NK cell-specific engager molecule using the extracellular domain of NKG2D as the target cell-binding domain and NKp46 as the NK cell-binding domain.

### CD16

CD16, also known as FcγRIII, is a glycoprotein with a molecular weight of 50,000 to 70,000 and a member of the Ig superfamily. It is mainly expressed on the surface of monocytes and natural killer cells and is involved in antibody-dependent cellular cytotoxicity (ADCC). In tumor therapy, activating NK cells using CD16 antibodies can promote NK cell infiltration and induce stronger NK cell-mediated ADCC. The present invention employs a multispecific antibody that binds to CD16 to activate and bridge NK cells, promoting the killing of senescent cells.

### IL15

Interleukin-15 (IL-15) is a T cell growth factor that activates JAK1/JAK3 and STAT3/STAT5, Syk kinase and phospholipase C (PLC) γ, Lck kinase and Shc, thereby activating the PI3K/Akt and Ras/Raf/MAPK signaling cascades. Literature indicates that IL-15 participates in regulating the survival, proliferation, and function of immune cells such as NK cells, memory CD8⁺ T cells, and NKT cells, thereby inhibiting tumorigenesis and development.

The present invention also constructs a multispecific cell engager based on NKG2D-NKp46 and comprises an IL-15 activation unit, in order to enhance NK cell-mediated killing of senescent cells.

### Antibody

The second binding domain of the cell engager molecule of the present invention may include an antibody or antigen-binding fragment that specifically binds to NKp46 and/or an antibody or antigen-binding fragment that specifically binds to CD16.

The term "antibody" or "immunoglobulin" as used herein refers to a heterotetrameric glycoprotein having the same structural feature of about 150,000 daltons consisting of two identical light chains (L) and two identical heavy chains (H). Each light chain is linked to a heavy chain by a covalent disulfide bond, and the numbers of disulfide bonds between the heavy chains of different immunoglobulin isoforms are different. Each heavy and light chain also has regularly spaced intrachain disulfide bonds. One end of each heavy chain has a variable region (VH) followed by a plurality of constant regions. There is a variable region (VL) at one end of each chain and a constant region at the other end; the constant region of the light chain corresponds to the first constant region of the heavy chain; the variable region of the light chain corresponds to the variable region of the heavy chain. An interface is formed between the variable regions of the light and heavy chains by particular amino acid residues.

As used herein, the term "variable" means that some certain portions of the variable region of an antibody differ in sequence and contribute to the binding and specificity of each particular antibody to its particular antigen. However, the variability is not evenly distributed throughout the antibody variable region. It is concentrated in three regions in the light and heavy chain variable regions called complementarity determining regions (CDRs) or hypervariable regions. The more conserved portions of the variable regions are referred as framework regions (FRs). The variable regions of the natural heavy and light chains each comprises four FR regions, which are in a substantially β-folded configuration, and are linked by three CDRs that form the linker ring and, in some cases, form a partial β-folded structure. The CDRs in each chain stand close together through FR regions and form the antigen-binding site of the antibody together with the CDRs of the other chain (see Kabat et al., NIH Publ. No. 91-3242, Vol. I, 647-669 (1991)). Constant regions are not directly involved in the binding of the antibodies to the antigens, but they exhibit different effector functions, such as antibody-dependent cellular cytotoxicity involved in antibodies.

The term "antibody fragment" or "antigen-binding fragment" refers to a portion of an antibody, such as F(ab')2, F(ab)2, Fab', Fab, Fv, single-chain Fv (scFv), single-chain antibody, disulfide-linked Fv (sdFv), fragment comprising a VL or VH domain, fragment produced by Fab expression library, and anti-idiotypic (anti-Id) antibody. Regardless of structure, antibody fragments bind to the same antigen recognized by the intact antibody. The term "antibody fragment" includes DARTs and diabodies. The term "antibody fragment" also includes any synthetic or genetically engineered protein comprising an immunoglobulin variable region that acts like an antibody by forming a complex with a specific antigen. "Single-chain fragment variable region" or "scFv" refers to a fusion protein of the variable regions of the heavy chain (VH) and light chain (VL) of an immunoglobulin. In some aspects, the domains are connected by a short linker peptide of 10 to about 25 amino acids. The linker may be rich in glycine for flexibility and serine or threonine for solubility and may connect the N-terminus of VH or the C-terminus of VL or vice versa. Despite the removal of the constant region and the introduction of a linker, such proteins retain the specificity of the original immunoglobulin. For IgG, a standard immunoglobulin molecule comprises two identical light chain polypeptides with a molecular weight of about 23,000 daltons and two identical heavy chain polypeptides with a molecular weight of 53,000-70,000. The four chains are typically connected by disulfide bonds in a "Y" configuration, where the light chains connect the heavy chains from bracket of the "Y" the and extend through the variable region.

As mentioned above, the variable region allows the antibody to selectively recognize and specifically bind to an epitope on an antigen. That is, the VL and VH domains of the antibody or a subset of the complementarity-determining regions (CDRs) combine to form a variable region that defines a three-dimensional antigen-binding site. This quaternary antibody structure forms the antigen-binding sites present at the ends of each arm of the Y configuration. More specifically, the antigen-binding site is defined by three CDRs on each of the VH and VL chains (i.e., HCDR1, HCDR2, HCDR3, LCDR1, LCDR2, and LCDR3). In some cases, such as certain immunoglobulin molecules derived from camelid species or engineered based on camelid immunoglobulins. Alternatively, the immunoglobulin molecule may consist of only heavy chains without light chains or only light chains without heavy chains.

As used herein, antibodies, antigen-binding fragments, or antibody domains also include their "variants", where "variants" refer to antibodies, antibody fragments, or antibody domains that: (1) have at least 80%, 85%, 90%, 95%, 96%, 97%, 98%, or 99% sequence identity to the original antibody, antibody fragment, or antibody domain, and (2) specifically bind to the same target as the original antibody, antibody fragment, or antibody domain. It should be understood that when sequence identity is expressed in the form of "at least x% identical" or "at least x% identity", such embodiments include any and all numerical percentages equal to or higher than the lower limit. Furthermore, it should be understood that when an amino acid sequence is present in this application, it should be interpreted as also disclosing or comprising an amino acid sequence with at least 80%, at least 85%, at least 90%, at least 95%, at least 96%, at least 97%, at least 98%, or at least 99% identity.

The antibody comprised in the cell engager molecule of the present invention may be an immunologically active antibody fragment, such as a Fab or (Fab')2 fragment; an antibody heavy chain; or an antibody light chain. The antibody used in the present invention is preferably in the form of a single-chain antibody (scFv), which contains the heavy chain variable region and the light chain variable region of an antibody but lacks the constant region, and is the smallest antibody fragment with complete antigen-binding sites. Generally, the Fv antibody also comprises a polypeptide linker between the VH and VL domains and is capable of forming the structure required for antigen binding.

The terms "specifically binds", "selectively binds", "selectively binding", and "specifically binding" refer to the binding of an antibody to an epitope on a predetermined antigen. Typically, the antibody binds with an affinity (KD) of less than about 10⁻⁷ M, for example, less than about 10⁻⁸ M, 10⁻⁹ M, or 10⁻¹⁰ M, or even lower.

As used herein, the term "heavy chain variable region" and "VH" can be used interchangeably.

As used herein, the term "light chain variable region" and "VL" can be used interchangeably.

As used herein, the term "variable region" and "complementarity determining region (CDR)" can be used interchangeably.

The term "CDR" refers to one of the six hypervariable regions within the variable domain of an antibody that primarily contributes to antigen binding. One of the most commonly used definitions of these six CDRs is provided by Kabat E.A. et al. (1991) Sequences of Proteins of Immunological Interest. NIH Publication 91-3242.

At the same time, those skilled in the art should understand that although the first binding domain of the BiTE of the present invention is preferably the extracellular domain of NKG2D, the first binding domain may also be selected as an antibody that specifically targets NKG2D ligands, as long as it can achieve the cell-binding effect of the present invention.

### Bispecific Cell Engager Molecule

As used herein, the terms "bispecific cell engager molecule", "bispecific cell engager", "cell engager", "bispecific antibody", and "BiTE" are used interchangeably and all refer to the cell engager molecule provided in the first aspect of the present invention, which is capable of simultaneously binding to a NKG2D ligand and NKp46.

Bispecific cell engager molecules are formed by connecting two proteins or polypeptide sequences (most commonly antibodies) that bind to different target proteins. The function of the bispecific cell engager molecule of the present invention is determined by the specific gene sequences of the extracellular domain of the NKG2D receptor and the NKp46 antigen-binding domain. The antibody of the present invention can simultaneously bind to NKG2D ligands and NKp46, connecting target cells expressing NKG2D ligands via the NKG2D extracellular domain and natural killer cells via the NKp46 binding domain, thereby effectively bridging natural killer cells with target cells and promoting the cytotoxic effects of natural killer cells.

As used herein, the term "bispecific" refers to a molecule containing at least two binding domains with different binding specificities. Each binding domain is capable of specifically binding to a target molecule. In some embodiments, the bispecific cell engager is a polymeric molecule with two or more peptides. In some embodiments, the binding domain comprises a single-domain antibody, an antigen-binding fragment of an antibody, a single-chain variable fragment, or a variable region, or CDR, or a combination thereof that specifically binds to a target protein. In some embodiments, the binding domain includes a ligand that specifically binds to the target protein, or a fragment thereof. In some embodiments, the binding domain comprises a combination of the above structures.

The at least two targeting domains of the cell engager molecule of the present invention are optionally connected by a linker peptide or single Fc fragment. A preferred linker peptide sequence is (G₄S)₃, but is not limited thereto.

In the present invention, the BiTE of the present invention further includes conservative variants thereof, which refer to polypeptides that comprises at most 10, preferably at most 8, more preferably at most 5, most preferably at most 3 amino acids replaced by amino acids with the same or similar properties compared with the amino acid sequence of the antibody of the present invention. These conservatively variant polypeptides are preferably produced by amino acid substitution according to Table 1.

**Table 1**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

Moreover, the amino acid sequences also include sequences formed by the addition, deletion, modification, and/or substitution of at least one amino acid, preferably sequences with homology or sequence identity of at least 80%, more preferably at least 85%, even more preferably at least 90%, and most preferably at least 95%.

Methods for determining sequence homology or identity well-known to those skilled in the art include, but are not limited to: Computational Molecular Biology, edited by Lesk, A.M., Oxford University Press, New York, 1988; Biocomputing: Informatics and Genome Projects, edited by Smith, D.W., Academic Press, New York, 1993; Computer Analysis of Sequence Data, Part I, edited by Griffin, A.M. and Griffin, H.G., Humana Press, New Jersey, 1994; Sequence Analysis in Molecular Biology, von Heinje, G., Academic Press, 1987; and Sequence Analysis Primer, edited by Gribskov, M. and Devereux, J., M Stockton Press, New York, 1991 and Carillo, H. and Lipman, D., SIAM J. Applied Math.48:1073(1988). The preferred method for determining identity shall maximize the alignment between the sequences being tested. Methods for determining identity are compiled into publicly available computer programs. Preferred computer programs for determining the identity between two sequences include, but are not limited to: the GCG package (Devereux, J. *et al.*, 1984), BLASTP, BLASTN, and FASTA (Altschul, S.F. *et al.*, 1990). The BLASTX program is publicly available from NCBI and other sources (BLAST Manual, Altschul, S. et al., NCBI NLM NIH Bethesda, Md. 20894; Altschul, S. *et al.*, 1990). The well-known Smith-Waterman algorithm can also be used to determine identity.

The antibody of the present invention may be a single-chain antibody or a bispecific antibody targeting NKG2D ligands and an immune cell membrane protein (e.g., human NKG2D ligands and NKp46).

In one preferred embodiment of the present invention, the bispecific cell engager molecule is a single-chain antibody comprising an anti-NKp46 single-chain antibody segment, a linker peptide, and the extracellular domain of NKG2D, wherein the anti-NKp46 single-chain antibody is a conventional single-chain antibody in the art, comprising a heavy chain variable region and a light chain variable region.

In the aforementioned context of the present invention, the number of added, deleted, modified, and/or substituted amino acids is preferably not more than 40% of the total number of amino acids in the initial amino acid sequence, more preferably not more than 35%, more preferably 1-33%, more preferably 5-30%, more preferably 10-25% and more preferably 15-20%.

In the aforementioned context of the present invention, more preferably, the number of amino acids added, deleted, modified, and/or substituted may be 1-7, more preferably 1-5, even more preferably 1-3, and most preferably 1-2.

### Recombinant Protein

The present invention also provides a recombinant protein, which comprises the cell engager molecule of the present invention.

The recombinant protein of the present invention may comprises monomers, dimers, or multimers of the cell engager molecule of the present invention; or multispecific (e.g., trispecific) cell engagers.

The preparation method of the recombinant protein is a conventional method in the field. The preferred preparation method is: isolating it from a recombinant expression transformant expressing the protein or obtaining it by artificial synthesis of the protein sequence. The method of isolation from a recombinant expression transformant preferably includes the following steps: cloning a nucleic acid molecule encoding the protein and containing point mutations into a recombinant vector, transforming the resulting recombinant vector into a transformant to obtain a recombinant expression transformant, and isolating and purifying the recombinant protein by culturing the resulting recombinant expression transformant.

### Nucleic Acid

The present invention also provides a polynucleotide molecule encoding the aforementioned cell engager molecule. The polynucleotides of the present invention can be in the form of DNA or RNA. DNA forms include cDNA, genomic DNA, or synthetic DNA. DNA can be single-stranded or double-stranded. DNA can be the coding strand or the non-coding strand. The coding sequence for a mature polypeptide can be the same as the coding sequence of the cell engager of the present invention or a degenerate variant thereof. As used herein, "degenerate variant" in the present invention refers to a nucleic acid sequence that encodes an amino acid sequence identical to the polypeptide of the present invention but differs in its coding sequence.

The polynucleotides encoding the mature polypeptides of the present invention comprise coding sequences encoding only the mature polypeptide; coding sequences of the mature polypeptide and various additional coding sequences; coding sequences (and optionally additional coding sequences) of the mature polypeptide and non-coding sequences.

The term "polynucleotide encoding a polypeptide" may include a polynucleotide that encodes the polypeptide, or a polynucleotide that also includes additional coding and/or non-coding sequences.

The present invention also relates to polynucleotides that hybridize to the sequences as described above and having at least 50%, preferably at least 70%, more preferably at least 80% identical between the two sequences. In particular, the present invention relates to polynucleotides that can hybridize to the polynucleotides of the present invention under stringent conditions. In the present invention, "stringent conditions" means: (1) hybridization and elution at lower ionic strength and higher temperature, such as 0.2 x SSC, 0.1% SDS, 60°C; or (2) hybridization adding a denaturant, such as 50% (v/v) formamide, 0.1% calf serum/0.1% Ficoll, 42 °C, or the like; or (3) hybridization only occurs when the identity between the two sequences is at least 90%, more preferably 95% or more. And the polypeptide encoded by the hybridizable polynucleotide has the same biological function and activity as the cell engager molecule according to the first aspect of the present invention.

The whole length of the nucleotide sequence or the fragment thereof of the cell engager molecule of the present invention can be obtained via PCR amplification, recombinant method or artificial synthesis. One feasible method is to synthesize relevant sequences by artificial method, especially when the fragment is short in length. Usually, several small fragments are synthesized first, and then are linked together to obtain a fragment with a long sequence. In addition, the coding sequence and the expression label (e.g. 6His) can be fused together to form a fusion protein.

### Vector and Host Cell

The present invention also provides a recombinant expression vector containing the aforementioned nucleic acid.

The recombinant expression vector mentioned herein can be obtained through conventional methods in the field, namely, by linking the nucleic acid molecule of the present invention to various expression vectors. The expression vector is a conventional vector in the field, as long as it can accommodate the aforementioned nucleic acid molecule. The vector preferably includes various plasmids, cosmids, phages, or viral vectors.

The present invention also provides a recombinant expression transformant comprising the aforementioned recombinant expression vector.

The preparation method of the recombinant expression transformant is a conventional method in the field, preferably by transforming the aforementioned recombinant expression vector into a host cell. The host cell is a conventional host cell in the field, as long as it can ensure stable self-replication of the recombinant expression vector and effective expression of the carried nucleic acid. Preferably, the host cell is *E. coli* TG1 or *E*. *coli* BL21 cells, or HEK-293T or CHO cells. The preferred recombinant expression transformant of the present invention can be obtained by transforming the aforementioned recombinant expression plasmid into the host cell. The transformation method is a conventional method in the field, preferably by chemical transformation, heat shock, or electroporation.

In a preferred embodiment of the present invention, the optional vectors include: pCDH, pTOMO, pGEM, pELNS, pMSGV, or a combination thereof.

In a preferred embodiment of the present invention, the optional host cells include: T cells, NK cells, or a combination thereof.

### Preparation of the Cell Engager Molecule

The preparation method of the DNA sequence of the cell engager molecule or its fragment of the present invention preferably involves fusing the coding sequences of the ligand segment and the antibody segment to form a single-chain antibody. Additionally, conventional techniques such as PCR amplification or genomic library screening can be used to obtain the sequence.

Once a relevant sequence is obtained, the relevant sequence can be obtained in bulk using a recombination method. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

At present, DNA sequences encoding the cell engager molecule of the invention (or fragments thereof, or derivatives thereof) can be completely obtained by chemical synthesis. The DNA sequence can then be introduced into a variety of existing DNA molecules (or vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as a mammal cell. Preferred cell comprises, but is not limited to, T cells.

In general, under conditions suitable for expression of the antibody according to the present invention, the host cell obtained is cultured. Then, the antibody according to the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The obtained cell engager can be characterized using conventional methods. For example, its binding specificity can be determined by immunoprecipitation or *in vitro* binding assays such as radioimmunoassay (RIA) or enzyme-linked immunosorbent assay (ELISA). Its binding affinity, for instance, can be assessed using Scatchard analysis as described by Munson et al., Anal. Biochem., 107:220 (1980).

The cell engager of the present invention can be expressed intracellularly or on the cell membrane, or be secreted out of the cell. If desired, recombinant proteins can be isolated and purified by various separation methods using their physical, chemical and other properties. These methods are well known to those skilled in the art. Examples of such methods include, but are not limited to, conventional renaturation treatments, treatment with a protein precipitant (salting-out method), centrifugation, osmosis cell disruption, super-treatment, ultracentrifugation, molecular sieve chromatography (gel filtration), adsorption chromatography, ion-exchange chromatography, high performance liquid chromatography (HPLC) and various other liquid chromatography techniques and combinations of these methods.

Conventional methods for preparing bispecific cell engager molecules in the field include the following two approaches:
1. First, the bispecific antibody gene is prepared using PCR, and the gene is then cloned into the expression vector pRB199 and transformed into *E. coli* strain BL21(λDE3) to prepare inclusion bodies. Subsequently, the inclusion bodies are denatured by adding 6M guanidine-HCl and DET (dithioerythritol), followed by a 100-fold dilution with refolding buffer, rapid mixed at 4°C, and incubated at 4°C for 72 hours to allow protein refolding. After refolding, dialysis is performed by adding 0.1M Tris and 0.5M NaCl at a 1:10 ratio, repeated three times, followed by filtration (0.2 µm) and metal ion affinity chromatography. Then the protein is purified using a fast protein liquid chromatography system (BioLogic DuoFlow 10 System; Bio-Rad) and isolated with a histidine-tagged protein purification column. The protein is eluted using a imidazole gradient at a flow rate of 1 mL/min. The product is subjected to column (Sartorius Stedim Biotech) processing to remove proteins with a molecular weight greater than 10,000, dialyzed with PBS, and filtered for sterilization. After concentration determination, the product is identified by silver staining using SDS/PAGE (see PNAS, 2013, 110(1): 270-275).
2. CHO cells are infected with lentivirus containing the bispecific antibody, and fluorescence expression is observed in CHO cells after 72 hours of culture. The successfully infected cell line is expanded. CHO cells stably expressing the bispecific antibody can continuously secrete it. The cell supernatant is collected for protein purification and concentration. Then the protein is purified using a fast protein liquid chromatography system (BioLogic DuoFlow 10 System; Bio-Rad) and isolated with a histidine-tagged protein purification column. The nickel column is equilibrated with five times the sample volume of equilibration buffer at a flow rate of 0.5-1 mL/min. After equilibration, the sample is passed through the nickel column at a flow rate of 0.5 mL/min. The nickel column is washed with five times the volume of equilibration buffer to remove background proteins until the absorbance of the eluate at 280 nm is 0. The target protein is eluted with imidazole at a flow rate of 0.5 mL/min. The protein is then concentrated using an ultrafiltration tube and the salt solution is replaced. After concentration determination, the product is identified by Western blot (see Oncoimmunology, 2015, 4(4): e989776).

Those skilled in the art can make conventional selections or equivalent modifications to the above methods to prepare or produce the bispecific cell engager molecule of the present invention.

### Antibody-Drug Conjugates (ADCs)

The term "antibody-drug conjugate (ADC)" used in the present invention refers to a conjugate formed by the cell engager molecule of the present invention and an effector molecule.

Typically, the antibody-drug conjugate comprises the cell engager molecule and an effector molecule, wherein the cell engager molecule is conjugated, preferably chemically conjugated to the effector molecule. Preferably, the effector molecule is a therapeutically active drug. In addition, the effector molecule may be one or more of a toxic protein, a chemotherapeutic drug, a small-molecule drug or a radionuclide.

The cell engager molecule of present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that forms a covalent bond between the effector molecule and the cell engager molecule, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on a cell engager molecule (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. A cell engager molecule can be conjugated to a functional agent to form a conjugate of the cell engager molecule-functional agent. A functional agent (e.g., a drug, a detection reagent, a stabilizer) is conjugated (covalently linked) to a cell engager molecule. A functional agent can be linked to a cell engager molecule either directly or indirectly via a linker.

Cell engager molecules can be conjugated to drugs to form antibody-drug conjugates (ADCs). Typically, an ADC comprises a linker between a drug and a cell engager molecule. The linker can be a degradable or non-degradable linker. Typically, the degradable linker is easily degraded in an intracellular environment, for example, the linker is degraded at the target site, thereby releasing the drug from the cell engager molecule. Suitable degradable linkers include, for example, enzyme-degradable linkers, including peptidyl-containing linkers that can be degraded by protease (e.g., lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g., linkers that are hydrolyzed at a pH of below 5.5, such as hydrazone linkers) and linkers that are degraded under reducing conditions (e.g., disulfide-bond linkers). A non-degradable linker typically releases a drug under conditions that the cell engager molecule is hydrolyzed by protease.

A drug may be any cytotoxic, cytostatic or immunosuppressive drug. In an embodiment, a cell engager molecule is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to a cell engager molecule.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA groove binding reagents, DNA alkylation reagents and tubulin inhibitors, and typical cytotoxic drugs include, for example, auristatins, camptothecins, duocarmycins, etoposides, maytansines and maytansinoids (e.g., DM1 and DM4), taxanes, benzodiazepines, or benzodiazepine containing drugs (e.g., pyrrolo[1,4]benzodiazepines (PBDs), indolinobenzodiazepines, and oxazolidinobenzodiazepines) and vinca alkaloids.

In the present invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, as described in Bioconjugation Technology (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

### Use for detection and the kit

The bispecific cell engager molecule of the present invention or ADC thereof can be used for detection, for example, for detection of samples to provide diagnostic information.

In the present invention, the specimens (samples) used include cells, tissue samples and biopsy specimens. The term "biopsy" used in the present invention shall include all kinds of biopsy known to those skilled in the art. Therefore, the biopsy used in the present invention may include, for example, resection samples of tumors, tissue samples prepared by endoscopic methods or organ puncture or needle biopsy.

The samples used in the present invention include fixed or preserved cells or tissue samples.

The present invention also provides a kit containing the cell engager molecule (or a fragment thereof) of the present invention. In a preferred embodiment of the present invention, the kit further includes a container, instructions for use, buffer, and the like. In a preferred embodiment, the cell engager molecule of the present invention can be immobilized on a detection plate.

### Pharmaceutical composition

The present invention also provides a composition. In a preferred embodiment, the composition is a pharmaceutical composition comprising the aforementioned cell engager or an active fragment thereof, or a fusion protein thereof, or an ADC thereof, or a corresponding immune cell, and a pharmaceutically acceptable carrier. Typically, these substances can be formulated in non-toxic, inert, and pharmaceutically acceptable aqueous carrier media, wherein the pH is generally about 5-8, preferably about 6-8, although the pH value may vary depending on the nature of the substance being formulated and the condition to be treated.

The formulated pharmaceutical composition can be administered via conventional routes, including but not limited to: intratumorial, intraperitoneal, intravenous, or topical administration. Typically, the route of administration for the pharmaceutical composition of the present invention is preferably injection or oral administration. The injection administration preferably includes intravenous, arterial, intramuscular, intraperitoneal, intradermal, or subcutaneous injection routes. The pharmaceutical composition is in various conventional dosage forms in the art, preferably in solid, semi-solid, or liquid forms, and can be aqueous solutions, non-aqueous solutions, or suspensions, more preferably tablets, capsules, granules, injections, or infusions.

The cell engager of the present invention can also be used for cell therapy by expressing the nucleotide sequence in the cell.

The pharmaceutical composition of the present invention is a pharmaceutical composition used for the prevention and/or treatment of a disease associated with abnormal expression or function of NKG2D ligands.

The pharmaceutical composition according to the present invention comprises a safe and effective amount (e.g. 0.001-99 wt %, preferably 0.01-90 wt %, preferably 0.1-80 wt %) of the cell engager molecule according to the present invention (or a conjugate thereof), or a safe and effective amount (1×10³-1×10⁸ cells/ml, preferably 1×10⁴-1×10⁷ cells/ml) of the engineered immune cell and a pharmaceutically acceptable carrier or excipient. Such carriers include (but are not limited to): saline, buffers, glucose, water, glycerol, ethanol, and a combination thereof. Pharmaceutical preparations should correspond to the administration modes. The pharmaceutical composition according to the present invention can be prepared in the form of an injection, for example, by a conventional method using physiological saline or an aqueous solution containing glucose and other adjuvants. A pharmaceutical composition, for example, an injection and a solution, should be prepared under aseptic conditions. The administration amount of an active ingredient is a therapeutically effective amount, for example, about 1 µg per kilogram of body weight to about 5 mg per kilogram of body weight daily. In addition, the polypeptide according to the present invention may also be used in combination with an additional therapeutic agent.

In a preferred embodiment of the present invention, the polypeptide of the present invention can be used in combination with other therapeutic and/or preventive agents for treating and/or preventing cancer and/or cancer metastasis.

In the present invention, preferably, the pharmaceutical composition of the present invention further comprises one or more pharmaceutically acceptable carriers. The pharmaceutically acceptable carrier is a conventional pharmaceutically acceptable carrier in the art, and can be any suitable physiologically or pharmaceutically acceptable excipient. The excipient is a conventional excipient in the art, preferably including pharmaceutically acceptable excipients, fillers, or diluents. More preferably, the pharmaceutical composition comprises 0.01% to 99.99% of the aforementioned protein and 0.01% to 99.99% of the pharmaceutically acceptable carrier, wherein the percentages are mass percentages in the pharmaceutical composition.

In the present invention, preferably, the dosage of the pharmaceutical composition is an effective amount, wherein the effective amount is an amount capable of alleviating or delaying the progression of a disease, degenerative condition, or injury condition. The effective amount can be determined on an individual basis and will be partially based on considerations of the condition to be treated and the desired outcome. Those skilled in the art can determine the effective amount by using the aforementioned factors on an individual basis and using no more than routine experimentation.

When a conjugate is used, a safe and effective amount of conjugate is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 20 mg per kilogram of body weight. Of course, a specific amount should also depend on the factors such as administration route and physical conditions of a patient, which fall into the skills of skilled physicians.

### Therapeutic application

The present invention provides the use of the NKG2D ligand-targeting cell engager molecule and the pharmaceutical composition of the present invention for the prevention and/or treatment of disease expressing NKG2D ligands.

Wherein the disease expressing NKG2D ligands includes tumors, autoimmune diseases, transplant rejection, inflammation, aging, and diseases associated with senescent cell accumulation.

Tumors expressing NKG2D ligands are selected from the group consisting of: lung cancer, ovarian cancer, colon cancer, liver cancer, gastric cancer, pancreatic cancer, kidney cancer, prostate cancer, breast cancer, bladder cancer, nasopharyngeal cancer, leukemia, lymphoma, glioblastoma, neuroblastoma, melanoma, and a combination thereof.

The diseases associated with senescent cell accumulation expressing NKG2D ligands is selected from the group consisting of: muscular atrophy, fatty liver, heart failure, atherosclerosis, diabetes, myocardial hypertrophy, osteoporosis, tissue/organ fibrosis, Alzheimer's disease, Parkinson's syndrome, arthritis, chronic obstructive pulmonary disease, and other degenerative diseases caused by cellular senescence, and a combination thereof.

Autoimmune diseases expressing NKG2D ligands are selected from the group consisting of: rheumatoid arthritis, colitis, celiac disease, multiple sclerosis, alopecia areata, type 1 diabetes, chronic obstructive pulmonary disease, atherosclerosis, and metabolic syndrome associated with type 2 diabetes.

The universal cell engager molecule of the present invention can also be used as a type of vaccine for *ex vivo* immunization and/or *in vivo* therapy of mammals. Preferably, the mammal is a human.

In addition to the use of cell-based vaccines for *ex vivo* immunization for cells, the present invention also provides compositions and methods for *in vivo* enhancing immune responses against target antigens in patients.

Pharmaceutical compositions of the present invention may be administered in a manner appropriate to the disease to be treated (or prevented). The quantity and frequency of administration will be determined by such factors as the character of the condition of the patient, and the type and severity of the patient's disease, although appropriate dosages may be determined by clinical trials.

When referring to "an immunologically effective amount", "an anti-tumor effective amount", "a anti-age effective amount", or "a therapeutic amount", the precise amount of the composition of the present invention to be administered can be determined by a physician, taking into account the individual differences in the patient's (subject's) age, weight, size of senescent tissue, degree of senescence, and condition.

### Main advantages of the present invention

The bispecific cell engager molecule constructed in the present invention simultaneously targets NKG2D ligands and NKp46 antigen. It can be directly infused into the body or carried by cells in the body (such as NK cells, T lymphocytes, CAR-NK cells, etc.) and continuously expressed in the body, thereby enabling the bispecific cell engager molecule to exert its cytotoxic effects *in vivo.* The main advantages include:
1) High Targeting Specificity: The bispecific cell engager molecule targeting cells with upregulated NKG2D ligand expression can effectively bridge target cells and natural killer cells, ensuring stable binding and strong cytotoxicity.
2) High safety: NKG2D ligands are important targets for natural immune cells to clear abnormal cells and tumor cells, and their expression on the surface of normal cells is strictly regulated. The NKG2D-NKG2D ligand signaling pathway has undergone long-term natural selection, ensuring high safety. Furthermore, a large number of clinical trials targeting NKG2D ligands are currently underway, and no serious treatment-related side effects have been observed.

The invention is further illustrated below in conjunction with specific embodiments. It should be understood that the examples are not intended to limit the scope of the invention. The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989), or in accordance with the conditions recommended by the manufacturer. Unless otherwise stated, percentages and parts are by weight.

The sequences involved in the Examples of the present invention are as shown in the following table.

**Table 2 Sequence listing**

| Name | Sequence | SEQ ID NO: |
|---|---|---|
| NKG2D extracellula r domain | FLNSLFNQEVQIPLTESYCGPCPKNWICYKNNCYQFF DESKNWYESQASCMSQNASLLKVYSKEDQDLLKLV KSYHWMGLVHIPTNGSWQWEDGSILSPNLLTIIEMQ KGDCALYASSFKGYIENCSTPNTYICMQRTV | 1 |
| NKp46 antibody Fab | QVQLVQSGAEVKKPGSSVKVSCKASGYTFSDYVIN WVRQAPGQGLEWMGEIYPGSGTNYYNEKFKAKATI TADKSTSTAYMELSSLRSEDTAVYYCARRGRYGLY AMDYWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTKV DKRVEPGGGGSGGGGSGGGGSDIQMTQSPSSLSASV GDRVTITCRASQDISNYLNWYQQKPGKAPKLLIYYT SRLHSGVPSRFSGSGSGTDFTFTISSLQPEDIATYFCQ QGNTRPWTFGGGTKVEIKRTVAAPSVFIFPPSDEQLK SGTASVVCLLNNFYPREAKVQWKVDNALQSGNSQE SVTEQDSKDSTYSLSSTLTLSKADYEKHKVYACEVT | 2 |
| | HQGLSSPVTKSFNR | |
| NKp46 antibody HCDR1 | GYTFSDYV | 3 |
| NKp46 antibody HCDR2 | IYPGSGTN | 4 |
| NKp46 antibody HCDR3 | ARRGRYGLYAMDY | 5 |
| NKp46 antibody LCDR1 | QDISNY | 6 |
| NKp46 antibody LCDR2 | YTS | / |
| NKp46 antibody LCDR3 | QQGNTRPWT | 7 |
| Heavy chain variable region of NKp46 antibody | QVQLVQSGAEVKKPGSSVKVSCKASGYTFSDYVIN WVRQAPGQGLEWMGEIYPGSGTNYYNEKFKAKATI TADKSTSTAYMELSSLRSEDTAVYYCARRGRYGLY AMDYWGQGTTVTVSS | 8 |
| Light chain variable region of NKp46 antibody | DIQMTQSPSSLSASVGDRVTITCRASQDISNYLNWYQ QKPGKAPKLLIYYTSRLHSGVPSRFSGSGSGTDFTFTI SSLQPEDIATYFCQQGNTRPWTFGGGTKVEIKR | 9 |
| NKp46 antibody scFv | QVQLVQSGAEVKKPGSSVKVSCKASGYTFSDYVIN WVRQAPGQGLEWMGEIYPGSGTNYYNEKFKAKATI TADKSTSTAYMELSSLRSEDTAVYYCARRGRYGLY AMDYWGQGTTVTVSSGGGGSGGGGSGGGGSDIQM TQSPSSLSASVGDRVTITCRASQDISNYLNWYQQKP GKAPKLLIYYTSRLHSGVPSRFSGSGSGTDFTFTISSL QPEDIATYFCQQGNTRPWTFGGGTKVEIKR | 10 |
| NKp46 V_{H}H antibody | QVQLVQSGAEVKKPGSSVKVSCKASGYTFSDYVIN WVRQAPGQGLEWMGEIYPGSGTNYYNEKFKAKATI TADKSTSTAYMELSSLRSEDTAVYYCARRGRYGLY AMDYWGQGTTVTVSSAS | 11 |
| CD16 V_{H}H antibody CDR1 | TLTFTPYR | 12 |
| CD16 V_{H}H antibody CDR2 | ISSGDGRTT | 13 |
| CD16 V_{H}H antibody CDR3 | NTFVSFVGIARS | 14 |
| CD16 V_{H}H antibody | EVQLVESGGGFVQAGESLTLSCTSSTLTFTPYRMAW YRQAPGKQRDLVADISSGDGRTTNYADFAKGRFTIS RDNIKNTVFLRMTNLKPEDTAVYYCNTFVSFVGIAR SWGQGTQVTVSS | 15 |
| Fc fragment of IgG antibody binding CD16 | PKSCDKTHTCPPCPAPELVGGPSVFLLPPKPKDTLMI SRTPEVTCVVVDVSHEDPEVKFNWYVDGVEVHNAK TKPPEEQYNSTLRVVSVLTVLHQDWLNGKEYKCKV SNKALPAPIEKTISKAKGQPREPQVYTLPPSRDELTK NQVSLTCLVKGFYPSDIAVEWESNGQPENNYKTTPL VLDSDGSFFLYSKLTVDKSRWQQGNVFSCSVMHEA LHNHYTQKSLSLSPGK | 16 |
| IL15 protein sequence | NWVNVISDLKKIEDLIQSMHIDATLYTESDVHPSCKV TAMKCFLLELQVISLESGDASIHDTVENLIILANNSLS SNGNVTESGCKECEELEEKNIKEFLQSFVHIVQMFIN TS | 17 |
| Scfc sequence (CH2-CH3-CH2-CH3) | APELLGGPSVFLFPPKPKDTLMTSRTPEVTCVVVDVS HEDPEVKFNWYVDGVEVHNAKTKPCEEQYNSTYRC VSVLTVLHQDWLNGKEYKCKVSNKALPAPIEKTISK AKGQPREPQVYTLPPSREEMTKNQVSLTCLVKGFYP SDIAVEWESNGQPENNYKTTPPVLDSDGSFFLYSKLT VDKSRWQQGNVFSCSVMHEALHNHYTQKSLSLSPG KGGGGSGGGGSGGGGSGGGGSGGGGSGGGGSGGG GSGGGGSGGGGSGGGGSGGGGSGGGGSGGGGSDK THTCPPCPAPELLGGPSVFLFPPKPKDTLMTSRTPEV TCVVVDVSHEDPEVKFNWYVDGVEVHNAKTKPCEE QYNSTYRCVSVLTVLHQDWLNGKEYKCKVSNKALP APIEKTISKAKGQPREPQVYTLPPSREEMTKNQVSLT CLVKGFYPSDIAVEWESNGQPENNYKTTPPVLDSDG SFFLYSKLTVDKSRWQQGNVFSCSVMHEALHNHYT QKSLSLSP | 18 |

The Fab sequence of the NKp46 antibody is cited from the literature *Multifunctional Natural Killer Cell Engagers Targeting NKp46 Trigger Protective Tumor Immunity.* The CD16 VHH antibody sequence is cited from GenBank ABS29544.1. The IgG antibody Fc fragment sequence is derived from mogamulizumab.

### Example 1 Preparation of NKG2D-NKp46 Protein

### 1.1 Vector Construction

The nucleotide sequence of the target gene with the structure shown in Fig. 1A was synthesized and cloned into the lentiviral vector pCDH-CMV-MCS-EF1-Puro (refer to Myeloid Leukemia. Mol Ther, 2016. 24(9): p. 1615-26.) through EcoR I and Swa I restriction sites. After verifying the correctness of the cloned vector through restriction enzyme digestion and sequencing, it was transformed into competent *E. coli* (Stbl3) and expanded for culture. The plasmid was then extracted using the QIAGEN endotoxin-free midi extracting kit and identified by Hind III restriction enzyme digestion.

### 1.2 Virus Package

HEK-293 T cells were cultured in a 15 cm culture dish for virus package. Transfection was carried out when the confluence of HEK-293T cells was about 90%. A plasmid mixture dissolved in 2ml OPTIMEM was prepared (core plasmid 20ug, pCMVΔR8.9 10ug, PMD2.G 4ug); and 2ml OPTIMEM and 68ul of lipo 8000 were combined in another tube. After set at room temperature for 5 minutes, the plasmid complex was added to the liposome complex and set at room temperature for 20 minutes. The mixture was then added dropwise to the HEK-293T cells and incubated at 37°C for 6 hours before removing the medium. Pre-warmed complete media were added. Viral supernatants were collected 48 hours and 72 hours after transfection, centrifuged at 3000 rpm for 20 minutes at 4 °C, filtered through a 0.45um filter membrane, and then concentrated by centrifugation at 25000rpm for 2.5 hours at 4 °C. The concentrated virus was dissolved overnight in 30 ul of virus dissolving solution, and the virus titer was detected by QPCR.

### 1.3 Protein Preparation and Purification

The CHO cells were infected with the aforementioned virus, and after 24 hours, 1 µg/mL puromycin was added for 48 hours of selection. After selection, the CHO cells were further cultured for 7 days. The culture supernatant was collected and filtered through a 0.22 µm membrane. The His-tagged antibody was obtained from the expression supernatant using an affinity chromatography column. The equilibration buffer was 20 mM sodium phosphate buffer containing 0.5 M NaCl (900 mL, pH 7.4). The elution buffer was 20 mM sodium phosphate buffer containing 0.5 M imidazole and 0.5 M NaCl (900 mL, pH 7.4). The NKG2D-NKp46 bispecific antibody was obtained by passing through a cation exchange column and was finally concentrated by buffer exchange with PBS. The SDS-PAGE electrophoresis of the purified NKG2D-NKp46 protein is shown in Fig. 1B. The Western Blot results shown in Fig. 1B indicate that the molecular weight matches the theoretical value.

### 1.4 Detection of NKG2D-NKp46 Binding to NK Cells and Target Cells

(1) NK cells or target cells were resuspended in 200 µL of 1x PBS (containing 2% FBS);
(2) The resuspended cells was added with NKG2D-NKp46 proteins at a final concentration of 100 µg/mL, mixed well, and incubated on ice for 120 minutes. Cells were subjected to vortex every 10 minutes during incubation. The cells were then centrifuged at 500 g for 5 minutes, and the supernatant was discarded;
(3) The cells were resuspended in 1 mL of 1x PBS (containing 2% FBS) and centrifuged at 500 g for 5 minutes;
(4) The step was repeated;
(5) The cells was added with anti-His antibody, mixed well, and incubated on ice for 60 minutes. Cells were subjected to vortex every 10 minutes during incubation. The cells were then centrifuged at 500 g for 5 minutes, and the supernatant was discarded;
(6) Fluorescently labeled goat anti-rabbit secondary antibody was added, and the cells were incubated at room temperature for 30 minutes, followed by centrifugation at 500 g for 5 minutes. The supernatant was discarded;
(7) The cells were resuspended in 1 mL of 1x PBS (containing 2% FBS) and centrifuged at 500 g for 5 minutes;
(8) The step 7 was repeated;
(9) The binding ratio was detected by flow cytometry. The results are shown in Figs. 1C, 2B and 4A. The results indicate that NKG2D-NKp46 can bind to NK cells and target cells.

### Example 2 NKG2D-NKp46 Protein Promotes the NK Cell-Mediated Killing of Tumor Cells

### 2.1 Detection of NKG2D Ligand Expression in Tumor Cells

(1) MHCC97H cells were collected by trypsin digestion, washed three times with 1 x PBS, and resuspended in 200 µL of 1x PBS (containing 2% FBS) to adjust the cell concentration to 1x10⁶ cells/mL.
(2) The resuspended cells was added with NKG2D ligand antibody, mixed well, and incubated on ice for 120 minutes. Cells were subjected to vortex every 10 minutes during incubation. The cells were then centrifuged at 500 g for 5 minutes, and the supernatant was discarded;
(3) The cells were resuspended in 1 mL of 1x PBS (containing 2% FBS) and centrifuged at 500 g for 5 minutes;
(4) The step was repeated;
(5) The expression of NKG2D ligands was detected by flow cytometry. The results, as shown in Fig. 2A, indicate a significant upregulation of MICA and ULBP2 expression in MHCC97H cells.

### 2.2 NKG2D-NKp46 Protein Promotes the NK Cell-Mediated Killing of Tumor Cells

MHCC97H cells were co-incubated with NK cells at an effector-to-target ratio of 1:5 (NK cells as effector cells at a concentration of 1*10⁵/mL, 100 µL per well; tumor cells as target cells at a concentration of 2*10⁴/mL, 100 µL per well), and 100 µg/mL of NKG2D-NKp46 protein was added, followed by co-incubation for 8 hours. Cell killing effect was detected using the Promega fluorescence detection kit. First, the cells were lysed with 30 ul of 1*PLB lysis buffer for 20 minutes. Then, 30 ul of substrate was added to each well, and the reaction was immediately detected using a BioTek plate reader. The cytotoxicity killing rate = 1 - the fluorescence value of target cells with effector cells / the fluorescence value of target cells without effector cells. As shown in Figure 2C, the addition of the NKG2D-NKp46 protein significantly enhanced the killing of tumor cells by NK cells. The supernatant from the culture system of 1ng/ml NKG2D-NKp46 was collected, and the IFN-γ concentration was detected using an ELISA kit. Statistical analysis was performed using GraphPad Prism software. The results are shown in Fig. 2D, indicating a significant increase in IFN-γ secretion after the addition of NKG2D-NKp46 compared to the control group.

### Example 3 Upregulation of NKG2D Ligand Expression in Senescent Cells

### 3.1 Construction of a Cell Senescence Model Overexpressing p16 Protein with Tet-on System

(1) 3 × 10⁵ cells were seeded in 10 cm dishes, and on the next day, the cell confluence reached approximately 20% after attachment;
(2) After cell attachment, the cells were infected with lentivirus overexpressing p16 protein using the Tet-on system at a multiplicity of infection (MOI) of 50-100. Polybrene (stock concentration: 8 mg/mL) was added at a ratio of 1:1000 to enhance infection efficiency;
(3) After 24 hours, the cells were subjected to a second infection with the same amount of virus;
(4) Four days after infection, the cells were selected with puromycin at a final concentration of 3 µg/mL;
(5) The constructed p16-overexpressing cells were passaged into well plates or dishes. After 24 hours of attachment, 1 µg/mL dox was added to induce p16 protein expression;
(6) After 8 days of induction, the cells were stained using the SA-β-gal staining kit (CS0030, Sigma) for detecting senescence. As shown in Fig. 3A, over 90% of the cells were positive, indicating that the cells had become senescent.

### 3.2 Detection of NKG2D Ligand Expression at the Transcriptional Level

(1) After preparing senescent cells as described above, 1~2 mL of Trizol was added to the 10 cm dishes based on cell density, and the dishes were placed on ice for 5 minutes. The cells were then mixed by pipetting;
(2) 1 mL of the lysate from each dish was transferred to a 1.5 mL EP tube, and 200 µL of chloroform was added. The mixture was shaken with force for 15 seconds and then placed at room temperature for 5 minutes, followed by centrifugation (4°C, 12,000 g, 15 minutes);
(3) 450 µL of isopropanol was added to a new EP tube;
(4) After centrifugation, the upper colorless layer was carefully transferred to the EP tube containing isopropanol, mixed, and incubated at room temperature for 10 minutes, followed by centrifugation (4°C, 12,000 g, 10 minutes);
(5) The supernatant was discarded, and the RNA was washed with 1 mL of 75% ethanol prepared with RNase-free water, followed by centrifugation (4°C, 7,500 g, 5 minutes);
(6) The supernatant was carefully discarded, and the tube was inverted to air dry for 5 minutes. Any remaining liquid on the tube walls was removed using a pipette tip;
(7) The RNA was dissolved in 30 µL of RNase-free water, immediately placed on ice, and its concentration was measured;
(8) Using the extracted RNA as a template, 2 µg of RNA was reverse transcribed into cDNA using the Thermo Scientific RevertAidTM First Strand cDNA Synthesis Kit. The reaction system was as follows:

**Table 3**

| Component | Amount |
|---|---|
| RNA Template | 2µg |
| Random Primer | 1 µL |
| RNase-free H₂O | ~12µL |
| Total volume | 12µL |

(9) The reaction mixture was added to a PCR tube according to the above system and placed in a PCR machine at 65 °C for 5 minutes, then immediately placed on ice. The following components were then added to the tube:

**Table 4**

| Component | Amount |
|---|---|
| 5x reaction buffer | 4µL |
| RiboLock RNase Inhibitor (20 U/µL) | 1µL |
| 10mM dNTP Mix | 2µL |
| RevertAid Reverse Transcriptase (200 U/µL) | 1µL |
| Total volume | 20µL |

The mixture was gently mixed and short spun, then placed in the PCR machine for the following reaction: 25°C for 5 minutes; 42°C for 1 hour; 70°C for 5 minutes;
(10) The expression of NKG2D ligands was detected by real-time quantitative PCR using the Thermo powerup^{™} SYBR Green Master Mix (A25742) kit according to the manufacturer's instructions. The program was as follows: 50°C for 2 minutes; 95°C for 2 minutes; 95°C for 15 seconds (40 cycles); 60°C for 1 minute (40 cycles); 12°C, forever;
(11) The data were exported in Excel format, and the relative expression levels of NKG2D ligands were calculated. As shown in Fig. 3B, the expression of both NKG2D ligands MICA and ULBP2 was significantly upregulated.

### 3.2 Detection of NKG2D Ligand Expression Level on Membrane

(1) HEIP-P16 cells were collected by trypsin digestion, washed three times with 1 x PBS, and resuspended in 200 µL of 1x PBS (containing 2% FBS) to adjust the cell concentration to 1x10⁶ cells/mL.
(2) The resuspended cells was added with NKG2D ligand antibody, mixed well, and incubated on ice for 120 minutes. Cells were subjected to vortex every 10 minutes during incubation. The cells were then centrifuged at 500 g for 5 minutes, and the supernatant was discarded;
(3) The cells were resuspended in 1 mL of 1x PBS (containing 2% FBS) and centrifuged at 500 g for 5 minutes;
(4) The step was repeated;
(5) The expression of NKG2D ligands was detected by flow cytometry. The results are shown in Fig. 3C.

### Example 4 NKG2D-NKp46 Protein Promotes the NK Cell-Mediated Killing of Senescent Cells

Senescent cells induced by P16 overexpression and NK cells were co-cultured in a 96-well plate at an effector-to-target ratio of 2:1 (senescent cells as target cells, NK cells as effector cells) for 8 hours. NKG2D-NKp46 protein was added to the experimental group and irrelevant protein was used as the control group. The number of live cells was counted under a microscope, and the T cell killing rate was calculated. Killing efficiency = (Number of target cells in the Blank group - Number of target cells in the co-culture group) / Number of target cells in the Blank group. The results are shown in Figs. 4B and 4C, demonstrating that compared to the control group, both NKG2D-NKp46 significantly enhance NK cell-mediated killing of senescent cells. The supernatant from the culture system of 1ng/ml NKG2D-NKp46 was collected, and the IFN-γ concentration was detected using an ELISA kit. Statistical analysis was performed using GraphPad Prism software. The results are shown in Fig. 4D, indicating a significant increase in IFN-γ secretion after the addition of NKG2D-NKp46 compared to the control group.

### Example 5 Protein Comprising NKp46 scFv Promotes NK Cell-Mediated Killing

Resting NK cells were co-cultured with P16 overexpression-induced senescent cells or the liver cancer cell line SMMC7721 at an effector-to-target ratio of 10:1 in a 96-well plate. Then the control protein, NKG2D-NKp46 scFv, NKG2D-scFc-NKp46 scFv, and NKG2D-scFc-NKp46 scFv-IL15 proteins were added respectively, whose structures are shown in Fig. 5A. The structure of scFc is: CH2-CH3-CH2-CH3. CH2 and CH3 are derived from human IgG1 antibody. NKp46 scFv is the anti-NKp46 single-chain antibody. After 24 hours of treatment, the number of live cells was counted under a microscope, and the NK cell killing efficiency was calculated. Killing efficiency = (Number of target cells in the Blank group - Number of target cells in the co-culture group) / Number of target cells in the Blank group. The results, as shown in Figs. 5B and 5C, demonstrate that the protein comprising NKp46 scFv significantly enhances the killing of target cells by resting NK cells compared to the control group. The attachment of IL15 further enhances this effect.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

## Claims

1. A cell engager molecule comprising:
(a) a first binding domain that specifically binds to a NKG2D ligand; and
(b) a second binding domain that specifically binds to NKp46.

2. The cell engager molecule according to claim 1, wherein the first binding domain is derived from an extracellular domain of NKG2D.

3. The cell engager molecule according to claim 1, wherein the amino acid sequence of the first binding domain is as shown in SEQ ID NO: 1; or is an amino acid sequence that has at least 95% sequence homology or sequence identity to the amino acid sequence as shown in SEQ ID NO: 1 and is capable of binding to a NKG2D ligand.

4. The cell engager molecule according to claim 1, wherein the second binding domain comprises an antigen-binding fragment specifically against NKp46.

5. The cell engager molecule according to claim 4, wherein the antigen-binding fragment against NKp46 comprises: an Fab fragment, a single-chain antibody (scFv), a single-domain antibody, and a combination thereof.

6. The cell engager molecule according to claim 5, wherein the Fab fragment and/or single-chain antibody against NKp46 comprises a heavy chain variable region and a light chain variable region, wherein the heavy chain variable region comprises the following heavy chain complementarity-determining regions (HCDRs):
1) HCDR1 with the amino acid sequence as shown in SEQ ID NO: 3;
2) HCDR2 with the amino acid sequence as shown in SEQ ID NO: 4;
3) HCDR3 with the amino acid sequence as shown in SEQ ID NO: 5; and
the light chain variable region comprises the following light chain complementarity determining regions (CDRs):
1) LCDR1 with the amino acid sequence as shown in SEQ ID NO: 6;
2) LCDR2 with the amino acid sequence YTS;
3) LCDR3 with the amino acid sequence as shown in SEQ ID NO: 7.

7. The cell engager molecule according to claim 5, wherein the heavy chain complementarity-determining regions of the anti-NKp46 single-domain antibody comprise:
1) HCDR1 with the amino acid sequence as shown in SEQ ID NO: 3;
2) HCDR2 with the amino acid sequence as shown in SEQ ID NO: 4;
3) HCDR3 with the amino acid sequence as shown in SEQ ID NO: 5.

8. The cell engager molecule according to claim 1, wherein the second binding domain further comprises: an antigen-binding fragment derived from an anti-CD16 antibody and/or a polypeptide of an IL15 protein.

9. A recombinant protein comprising the cell engager molecule according to any one of claims 1-8.

10. A polynucleotide which encodes a polypeptide selected from the group consisting of:
(1) the cell engager molecule according to any one of claims 1-8; or
(2) the recombinant protein according to claim 9.

11. A vector comprising the polynucleotide according to claim 10.

12. A engineered host cell, wherein the host cell comprises the vector according to claim 11 or has the polynucleotide according to claim 10 integrated into its genome.

13. An antibody conjugate which comprises:
(a) an antibody moiety, which is selected from the group consisting of the cell engager molecule according to claim 1; and
(b) a coupling moiety coupled to the antibody moiety, which is selected from the group consisting of a detectable label, a drug, and a combination thereof.

14. A pharmaceutical composition which comprises:
(a) an active ingredient selected from the group consisting of: the cell engager molecule according to claim 1, the recombinant protein according to claim 9, the host cell according to claim 12, the antibody conjugate according to claim 13, and a combination thereof; and
(b) one or more pharmaceutically acceptable carriers, diluents, fillers, binders, excipients, or a combination thereof.

15. Use of the cell engager molecule according to the claim 1, the recombinant protein according to claim 9, the host cell according to claim 12, the antibody conjugate according to claim 13, and/or the pharmaceutical composition according to claim 14 in the preparation of a medicament for treating a disease associated with upregulated NKG2D ligand expression.

16. The use according to claim 15, wherein the upregulation means that the ratio of the expression level of NKG2D ligand (F1) to that in normal cells and tissues (F0) (i.e., F1/F0) is ≥1.5, preferably ≥2, more preferably ≥2.5.

17. The use according to claim 15, wherein the NKG2D ligand is selected from the group consisting of: MICA, MICB, ULBP1, ULBP2, ULBP3, ULBP4, ULBP5, ULBP6, and a combination thereof.

18. The use according to claim 15, wherein the disease includes tumors, autoimmune diseases, transplant rejection, inflammation, aging, and diseases associated with senescent cell accumulation.

19. The use according to claim 18, wherein the tumor is selected from the group consisting of: acute myeloid leukemia (AML), multiple myeloma (MM), chronic lymphocytic leukemia (CLL), acute lymphoblastic leukemia (ALL), diffuse large B-cell lymphoma (DLBCL), lung cancer, ovarian cancer, colorectal cancer, liver cancer, gallbladder cancer, biliary tract cancer, gastric cancer, pancreatic cancer, kidney cancer, prostate cancer, breast cancer, bladder cancer, nasopharyngeal cancer, non-small cell lung cancer, glioblastoma, neuroblastoma, melanoma, and a combination thereof.

20. The use according to claim 18, wherein the disease associated with senescent cell accumulation is selected from the group consisting of: muscular atrophy, fatty liver, heart failure, atherosclerosis, diabetes, myocardial hypertrophy, osteoporosis, tissue/organ fibrosis, Alzheimer's disease, Parkinson's syndrome, arthritis, chronic obstructive pulmonary disease, and other degenerative diseases caused by cellular senescence, and a combination thereof.
